# EUROPEAN PATENT SPECIFICATION

(11) **EP 3 065 714 B1**
(45) Date of publication and mention of the grant of the patent: **12.02.2020**
(21) Application number: 14793168.7
(22) Date of filing: 05.11.2014
(51) Int. Cl.: A61K 9/16, A61K 47/26, A61K 47/36, A61K 47/38, A61K 31/787, A61P 31/16

(54) **POLYINOSINIC-POLYCYTIDYLIC ACID (POLY (I:C)) FORMULATIONS FOR THE TREATMENT OF UPPER RESPIRATORY TRACT INFECTIONS**
POLYINOSIN-POLYCYTIDYL-SÄURE (POLY(I:C))-FORMULIERUNGEN ZUR BEHANDLUNG VON INFEKTIONEN DER OBEREN ATEMWEGE
FORMULATIONS D'ACIDE POLYINOSINIQUE-POLYCYTIDYLIQUE (POLY (I:C)) POUR LE TRAITEMENT DES INFECTIONS DES VOIES RESPIRATOIRES SUPÉRIEURES

(30) Priority: 06.11.2013 EP 13191742
(43) Date of publication of application: 14.09.2016
(73) Proprietor: Janssen Sciences Ireland Unlimited Company, Co Cork (IE)
(72) Inventor: KLINGELEERS, Didier Mario Lodewijk, B-3511 Stokrooie (BE); VAN DIJCK, Alex Henri, B-3920 Lommel (BE); MENSCH, Jurgen, B-2430 Laakdal (BE); MALCOLM, Bruce Albert, Burnaby BC V5H 4G3 (CA)
(74) Representative: Daelemans, Frank F.R.
(86) International application number: PCT/EP2014/073762
(87) International publication number: WO 2015/067632

(56) References cited:
- WO-A1-2013/164380
- CN-A- 101 491 503
- CN-A- 101 757 018
- CHRISTOPHER M E ET AL: "Use of toll-like receptor 3 agonists against respiratory viral infections", ANTI-INFLAMMATORY & ANTI-ALLERGY AGENTS IN MEDICINAL CHEMISTRY, BENTHAM SCIENCE PUBLISHERS LTD, BUSSUM, NL, vol. 10, no. 5, 1 October 2011 (2011-10-01), pages 327-338, XP002681043, ISSN: 1871-5230, DOI: 10.2174/187152111800194434

## Description

The present invention relates to a microparticle consisting of polyinosinic acid, polycytidylic acid, pea starch, and water, a composition comprising a plurality of such microparticles, and the composition for use in treating and/or preventing infections or the common cold and a device, preferably a nasal delivery system, comprising said composition for use by a patient in need to prevent and/or treat infections or the common cold.

The common cold (also known as nasopharyngitis, acute viral rhinopharyngitis, acute coryza, or a cold) is a viral infectious disease of the upper respiratory system caused primarily by viruses.

### Viruses

The common cold is a viral infection of the upper respiratory tract. The most commonly implicated virus is the rhinovirus (30-50%), a type of picornavirus with 99 known serotypes. Others include coronavirus (10-15%), influenza (5-15%), human parainfluenza viruses, human respiratory syncytial virus, adenoviruses, enteroviruses, and metapneumovirus.

In total over 200 serologically different viral types cause colds. Coronaviruses are particularly implicated in adult colds. Of over 30 coronaviruses, 3 or 4 cause infections in humans, but they are difficult to grow in the laboratory and their significance is thus less well-understood. Due to the many different types of viruses and their tendency for continuous mutation, it is impossible to gain complete immunity to the common cold.

### Clinical Signs and Symptoms

The first indication of an upper respiratory virus is often a sore or scratchy throat. Other common symptoms are runny nose, congestion, and sneezing. These are sometimes accompanied by conjunctivitis (pink eye), muscle aches, fatigue, malaise, headache, weakness, or loss of appetite. Cough and fever generally indicate influenza rather than an upper respiratory virus with a positive predictive value of around 80%. Symptoms may be more severe in infants and young children, and in these cases it may include fever and hives. Upper respiratory viruses may also be more severe in smokers.

Viral replication begins 2 to 6 hours after initial contact. Symptoms usually begin 2 to 5 days after initial infection but occasionally occur in as little as 10 hours. Symptoms peak 2-3 days after symptom onset, whereas influenza symptom onset is constant and immediate. There is currently no known treatment that shortens the duration; however, symptoms usually resolve spontaneously in 7 to 10 days, with some symptoms possibly lasting for up to three weeks. In children the cough lasts for more than 10 days in 35-40% and continues for more than 25 days in 10% of the cases. The common cold is the most frequent infectious disease in humans with the average adult contracting two to four infections a year and the average child contracting several infections per year between 6-12 years of age. In the United States, the incidence of colds is higher in the fall (autumn) and winter, with most infections occurring between September and April. The seasonality may be due to the start of the school year or due to people spending more time indoors (in closer proximity with each other) increasing the chance of transmission of the virus.

### Infectious period

The common cold is most infectious during the first two to three days of symptoms however it is also infectious for a couple of days before the onset of symptoms and may still be somewhat infectious until symptoms have completely resolved.

### Human rhinovirus

Human rhinovirus is a member of the Enterovirus genus in the *Picornaviridae* family. The HRV particle is comprised of a 27-30 nm non-enveloped capsid consisting of 4 polypeptides (VP1, VP2, VP3, and VP4). The virus capsid contains a single-stranded RNA genome of approximately 7200 bases. A virally-encoded protein (VPg) is covalently attached to the 5' end of the RNA genome. The clinical course of infection with human rhinovirus (HRV) has been well characterized. HRVs can infect the upper and lower airways, nasal mucosa, sinuses and middle ear, and infections produce symptoms of "the common cold" (see above). Infections are self-limiting and are typically restricted to the upper airways. Peripheral white blood cell counts may be elevated during the first 2-3 days of the infection.

HRV infection can also lead to infection of the lower airways, otitis media (particularly in young children), and sinusitis. Serious complications (such as pneumonia) from rhinovirus infection are rare and have been reported to occur in infants and young children, particularly those with underlying conditions such as bronchopulmonary dysplasia, congenital heart disease, prematurity, and neurologic conditions, and immunosuppressed (bone marrow transplant recipients) adults. While other members of the *Picornaviridae* family can infect the central nervous system (i.e., poliovirus, enterovirus), infection of the human central nervous system by HRVs has not been reported.

### Treatment

There are no commercial antiviral agents for the treatment of rhinovirus infections or prevention of common colds. Treatment of upper respiratory tract infections caused by rhinoviruses are based upon management of the symptoms (sneezing, nasal congestion, rhinorrhea, eye irritation, sore throat, cough, headaches, fever, chills) typically using over the counter antihistamines, aspirin, cough suppressants, and nasal decongestants. More serious complications of HRVs infection (e.g. pneumonia) are managed using medically appropriate standards of care.

### Cost and Medical Need

According to data of the World Health Organization more than 1 billion cases of common cold were reported in the USA last year. In the United States, the common cold leads to 75 to 100 million physician visits annually at a conservative cost estimate of $7.7 billion per year. Americans spend $2.9 billion on over-the-counter drugs and another $400 million on prescription medicines for symptomatic relief. An estimated 22 to 189 million school days are missed annually due to a cold. As a result, parents missed 126 million workdays to stay home to care for their children. When added to the 150 million workdays missed by employees suffering from a cold, the total economic impact of cold-related work loss exceeds $20 billion per year. This accounts for 40% of time lost from work.

Airway epithelial cells are the primary target of upper respiratory tract (URT) infective agents like rhino-and corona viruses. As infection with these viruses occurs prior to the onset of symptoms that reflects immune system clearance of infected cells, direct antiviral therapeutic intervention is unlikely to prove very effective. In addition, realizing and sustaining active levels of direct anti-viral compounds in the nasal mucosa is very difficult due to its high turnover. Prophylaxis on the other hand, by exploiting the body's own defenses and inducing an anti-viral state in the nasal epithelial cells, has already been shown to result in significant protection against a subsequent viral challenge as well as to lower the disease-related symptoms.

Although colds may last only a week or two, severe colds can last for up to a month. Adults average two to three colds per year and children six to ten, depending on their age and exposure. There are hundreds of different serotypes of the cold virus, making it impossible to develop a standard vaccine prophylaxis that would be effective against all of them.

Symptomatic treatment generally involves using sleep-inducing oral antihistamines and /or vaso-constrictive decongestants that have stimulant side-effects. This is only marginally effective and these side-effects are often as debilitating as the infection itself. Although prevention would be the ideal solution, for the reasons cited above the chances of a broadly effective vaccine against all the different serotypes is highly unlikely in the near future. So, short of quarantine, people will be exposed to these infectious agents on a regular basis, especially during "cold season" and so a broadly effective, convenient, side-effect free prophylactic would have a major impact on public health and productivity in the work place.

Targeting the innate immune response, an "early warning system" for the body would solve the above mentioned issues. This system, present in nasal epithelial cells, once stimulated appropriately, leads the cells to think they are being attacked by a virus and triggers an anti-viral defense response. Once this happens, the cells are refractory to subsequent viral attack. Although some early work had been done in the late 1980's, looking at the use of immune stimulating molecules such as interferon to trigger an innate immune response, manufacture was expensive and their effects difficult to control.

CN 101757018 A discloses a polyinosinic-polycytidylic acid dry powder for livestock and poultry.

The goal of the current investigation has been to develop a formulation of a triggering molecule (Poly (I:C)) that can be used in a measurable and controllable fashion, for example, every couple of days or even once a week, to prime the innate immune system and provide protection against viral infection. The approach outlined below takes an existing agent, Poly (I:C), which has demonstrated efficacy, but which is impractical and renders it convenient and effective using formulation sciences.

Toll-like receptor 3 (TLR3) is a protein that in humans is encoded by the *TLR3* gene. TLR3 is a member of the Toll-like receptor family of pattern recognition receptors of the innate immune system which plays a fundamental role in pathogen recognition and activation of innate immunity. TLRs are highly conserved from Drosophila to humans and share structural and functional similarities. They recognize pathogen-associated molecular patterns (PAMPs) that are expressed on infectious agents, and mediate the production of cytokines necessary for the development of effective immunity. The various TLRs exhibit different patterns of expression. This TLR3 receptor is also expressed by airway epithelial cells and is restricted to the dendritic subpopulation of the leukocytes.

TLR3 recognizes double-stranded RNA (dsRNA). Double-stranded RNA is RNA with two complementary strands that can be formed during the viral replication cycle. Upon recognition, TLR 3 induces the activation of transcription factors like NF-kB and Interferon Regulatory Factor 3 (IRF3) to increase production of type I interferon which signal other cells to increase their antiviral defenses.

The structure of TLR3 forms a large horseshoe shape that contacts with a neighboring horseshoe, forming a "dimer" of two horseshoes. Much of the TLR3 protein surface is covered with sugar molecules, making it a glycoprotein, but on one face (including the proposed interface between the two horseshoes), there is a large sugar-free surface. This surface also contains two distinct patches rich in positively-charged amino acids, which may be a binding site for negatively-charged double-stranded RNA.

Polyinosine-polycytidylic acid (Poly (I:C)) is a double stranded RNA molecule with a MW distribution up to, for instance 3.600.000 Daltons. Poly (I:C) is a Toll Like Receptor 3 (TLR3) ligand that mimics viral RNA and is a known stimulant of the innate immune response. When administered nasally it induces expression of anti-viral proteins like Interferon α and β in the nasal epithelium. It has been demonstrated to reduce the number and severity of rhinovirus infections.

Poly (I:C) is usually an unstable molecule in normal aqueous solutions. Currently, to achieve an effective therapeutic or prophylactic effect, Poly (I:C) needs to be re-dissolved immediately prior to use and administered every 2 hours. To improve patient compliance and reduce the frequency of dosing, a novel formulation has been developed that is stable and shows enhanced efficacy.

Poly (I:C) has been formulated with several bioadhesive polymers that can prolong the residence time on the nasal epithelium and provide a more effective and controllable stimulation of the innate immune system.

The current invention provides the identification of a unique formulation that could be stored almost indefinitely at room temperature and which retains its innate immune system-stimulating activity.

The current inventive formulation contains water-soluble carriers and has the advantage of low viscosity characteristics.

When pea starch is used in the formulation of the composition comprising Poly (I:C), such composition shows surprisingly a less sticky behavior to the inside of a vial, tube or device (like sprays) when such vials, tubes or devices must be filled with the inventive composition compared to any other starch used for the same purpose. The technical advantage is that more precisely the dosage of said Poly (I:C) can be administered to the patient in need, since less composition will stick to the inner side of the nasal spray device accordingly. In addition the formulation enhances the efficacy of Poly (I:C) and permits much less frequent dosing with even greater TLR3 stimulating activity.

The invention therefore relates to a microparticle consisting of polyinosinic acid, polycytidylic acid, pea starch and water, and a composition comprising a plurality of such microparticles. Micro particles are particles with an average particle size between 0.1 µm and 100 µm. The carrier polymer is starch obtained from the plant genus Lathyrus more specifically from peas. Poly (I:C)-carrier-polymer microspheres, or also so-called micro particles, comprised in the composition are produced by means of a particle formation process such as a spray-dry process.

The ratio Poly (I:C) / pea starch according to the invention ranges from 1/200 (w/w) to 1 / 0.1 (w/w), but preferably from 1/100 (w/w) to 1/1 (w/w) and even more preferably from 1/100 (w/w) to 1/5 (w/w) while a ratio Poly (I:C) / pea starch between 1/12 and 1/9 (w/w) is most preferred. The same ranges and ratios apply for the other carriers mentioned according to the invention.

The Dᵥ50 (= volume based 50% cumulative undersize of the particle) of the micro particle in the composition according to the invention ranges from 0.1 micrometer to 200 micrometer, preferably from 1 micrometer to 50 micrometer, more preferably from 2 micrometer to 40 micrometer, even more preferably from 2 micrometer to 20 micrometer, and most preferred from 10 micrometer to 20 micrometer.

The composition of the invention can also be a liquid composition comprising an organic solvent, wherein the organic solvent is based on glycerol or ethanol or a combination thereof.

The composition of the invention can be used in human and/or animal medicine preferably for use in preventing and/or treating viral infections of the human upper respiratory tract such as what are referred to as "common colds". For animal use the composition according to the invention can be used as aerosol formulation in for instance stables, barns, chicken flocks and the like. The current composition can be used by patients suffering from asthma and/or COPD (Chronic Obstructive Pulmonary Disease) in order to potentially prevent and/or treat upcoming common cold symptoms.

A preferred way to prevent and/or treat upper respiratory infections is performed by nasal administration.

The composition of the current invention comprising micro particles of polyinosinic-polycytidylic acid (Poly (I:C)) and as a carrier polymer pea starch can be used for the treatment and/or prevention of (viral) infections or common cold, wherein the composition is administered by nasal application at a time interval that is in the range of one day to one month, more preferably from every couple of days or even once a week.

The above mentioned composition wherein the ratio Poly (I:C) / pea starch ranges from 1/200 (w/w) to 1 / 0.1 (w/w), but preferably from 1/100 (w/w) to 1/1 (w/w) and even more preferably from 1/100 (w/w) to 1/5 (w/w) while a ratio Poly (I:C) / pea starch between 1/12 and 1/9 (w/w) is most preferred, in combination with the micro particle size in the composition ranging from 0.1 micrometer to 200 micrometer, preferably from 1 micrometer to 50 micrometer, more preferably from 2 micrometer to 40 micrometer, even more preferably from 2 micrometer to 20 micrometer, and most preferred from 10 micrometer to 20 micrometer can be used for the treatment and/or prevention of (viral) infections or common cold, wherein said composition is administered by nasal application at a time interval that is in the range of one day to one month, more preferably from every couple of days or even once a week.

Part of the invention is also a device, in particular a nasal delivery system, comprising a composition according to the invention.

According to the invention, Poly (I:C) is formulated as a dry powder for nasal administration. To improve stability, Poly (I:C) is spray dried from an aqueous mixture containing pea starch and Poly (I:C).

Starch is believed to have a dual function: (1) to act as a bio-adhesive in the nose, (2) and to serve as protective matrix for stabilizing Poly (I:C). Starch, especially pea starch, is a preferred excipient for nasal application as accumulation is prevented by degradation through amylases.

Starches with high amylopectin content or with chemically modified starches exhibit good muco-adhesion. Specifically hydroxypropylated pregelatinized pea starch (chemically modified) was used in the present invention, as it is cold water-swelling and contains a cold water-soluble fraction, resulting in a homogeneous dispersion when mixed at low shear with Poly (I:C). The resulting starch dispersions have a low to medium viscosity which allows spray drying into a homogeneous powder.

Nasal administration is preferably achieved using a single dose nasal powder device (Unit dose device supplied from Aptar Pharma Germany). The unit dose device is an active delivery system, meaning that the patient does not need to inhale and performance is patient independent. Dosing is performed by actuation, which is controlled by overpressure. The dose per puff is determined by the concentration of Poly (I:C) in the spray dried powder and the emitted weight of the powder. The powder will be administered into each nostril using a new device for each puff.

As mentioned above Poly (I:C), is a synthetic double-stranded RNA composed of anti-parallel polynucleotide strands of inosinic acid and cytidylic acid sodium salts. The strands are non-covalently bound by hydrogen bonds formed between the inosine and cytosine bases.

The average chain length for the Poly (I:C) ranges between 300 to 6,000 base pairs, corresponding to approximately 180,000 to about 3,600,000 daltons. The molecular formula is (C₁₀H₁₀N₄NaO₇P)ₓ • (C₉H₁₁NaN₃O₇P)ₓ.

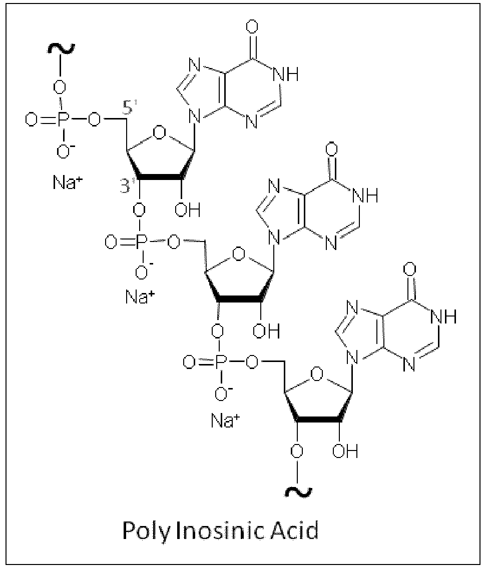

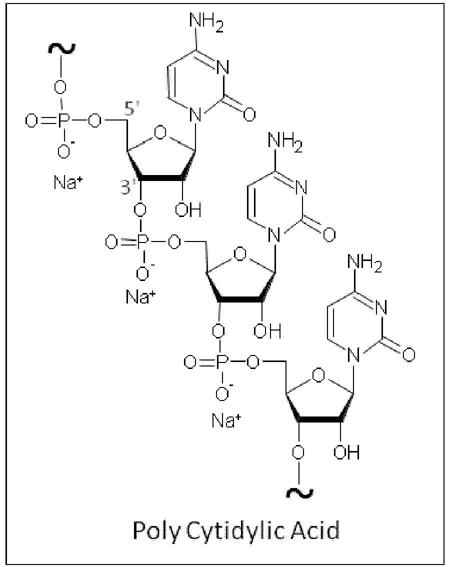

Above Poly (I:C) can be purchased, but can optionally also be made in house using for instance the following procedure

The duplex product Poly (I:C) is manufactured from the individual homopolymers Poly Inosine (I) and Poly Cytidine (C). Poly I and Poly C are synthesized by individually polymerizing the nucleoside diphosphates inosine and cytidine in the presence of polynucleotide phosphorylase (PNPase). Each nucleoside diphosphate is individually polymerized by PNPase for a 20-24 hrs. to control the length of the resulting ribonucleic acid polymer. The enzyme, protein kinase, is then added to terminate the polymerization reaction. The resulting homopolymers (i.e. single stranded RNA molecules) are hydrolyzed to control the molecular weight range of each polymer product within a specified range. The hydrolyzed product is treated with ethanol to precipitate the single stranded RNA molecules (ssRNA) from solution. The precipitate is separated from the supernatant and dissolved in water. The solution of ssRNA is then filtered to remove particulates, ultra filtered to remove the low- molecular weight contaminants and then lyophilized. Lyophilized ssRNA products are individually tested for purity, molecular weight, and other quality attributes to ensure the products are within specification.

The individual single stranded homo-polymers (Poly I and Poly C) are individually dissolved in 0.015 M sodium chloride and then combined to anneal the strands forming the double stranded duplex product (Poly I : Poly C). After mixing, the resulting solution is filtered. The filtrate is ultra-filtered to remove low molecular weight contaminants. The ultra-filtered product is then lyophilized. The resulting duplex product is stored at -20 °C. The lyophilized dsRNA product is tested for purity, molecular weight, and other quality attributes to ensure the product is within specification.

### Materials & Methods

### Spray drying of Poly (I:C) with carrier polymers

The spray dry process with the carriers according to the invention selected from the group pea starch (according to the invention), pregelatinized potato starch, lactose, microcrystalline cellulose, hyaluronate or glucosamine (all not according to the invention) was performed on a Buchi B290 Mini spray dryer (Buchi, Flawil, Switzerland). Demineralized water was filtered using a 0.2 micron cellulose acetate filter (Whatman FP30/0.2 CA-S) and added to a glass beaker. The excipients were added while stirring using a magnetic stirrer. When completely dissolved, Poly (I:C) was added to the solution. A total solids concentration of 0.5 % (w/w) and a ratio of Poly (I:C) /excipient 1/9 (w/w) was applied.

### Spray drying of additional Poly (I:C) - pea starch

The spray dry process was performed on a Buchi B290 Mini spray dryer (Buchi, Flawil, Switzerland). Nuclease free water added to a glass beaker and the pea starch is added while mixing using an Ultra Turax T25 (Janke &Kunkel), until the starch is completely dispersed. Poly (I:C) was dissolved in Nuclease free water and stirred on a magnetic stirrer until the Poly (I:C) is completely dissolved. The dissolved Poly (I:C) is added to the dispersed pea starch and stirred at room temperature, the Poly (I:C) solution is prepared just before spray drying. A total solids concentration of 4.5% (w/w), 10 % (w/w) or 20% (w/w) and a ratio of Poly (I:C) / pea starch 1/12 (w/w) was applied.

The solutions were fed to a two-fluid nozzle (diameter: 0.7 mm) at the top of the spray dryer by means of a peristaltic pump. The spray dryer operated in cocurrent nitrogen flow mode. The spray dried particles were collected in a reservoir attached to a cyclone. After collection of the particles, the glass cylinder and cyclone was cooled to room temperature. The collected powder was transferred to amber glass bottle and this bottle is placed in an aluminum vapor lock bag. The vials were stored at room temperature.

The same above process was used for spray-drying the other carriers (not according to the invention) wherein a ratio Poly (I:C) / carrier of 1/9 was applied.

### Scanning Electron Microscopy

The samples were sputtered with gold particles with diameter +/- 30-50nm. Images were generated using a FEI scanning electron microscope -type Quanta 200F with Everhart Thornley detector.

### Water content- Karl Fischer titration

Water content of the concepts was determined by means of a direct volumetric Karl Fisher titration. A KF TITRATOR V30 is used (Mettler Toledo, US). The powder (50-100 mg) was transferred to the titration vessel containing Hydranal® Methanol Dry (Sigma Aldrich) and stirred for 300 seconds. Titration was performed with Hydranal® Composite 2 (Sigma Aldrich) at a concentration of 2 mg/ml using a 5 ml burette. For termination a stop drift of 15 µg/min was applied. Samples were analyzed in triplicate.

### Determination of particle size

There exists a tendency to evaluate particle size distribution data merely on the basis of the volume distribution of the products of interest. Thereby, one often limits the valuation to a comparison of the Dᵥ10, Dᵥ50 and Dᵥ90 cumulative undersizes.

However, comparing dᵥx cumulative undersizes may not always be straightforward due to the fact that different techniques and instruments readily lead to different results.

In addition, one can get more information out of a particle size (or shape) distribution data by looking from a different perspective to the data (i.e., using other parameters).

For the determination of the particle size distribution the laser diffraction test method was used.

The analysis was performed on a Malvern Mastersizer 2000 laser diffractometer equipped with a Hydro2000S wet dispersion module (or an equivalent system). The instrument is used in the blue light ON detection mode at a size range of 20nm to 2mm.

### In vivo testing of formulations in the influenza mouse model

All animal studies were approved by the ethical committee and performed according to national and international guidelines. 8-12 week old female Swiss mice (Janvier) were used. All intranasal treatments were performed under isoflurane anesthesia. To administer an amount of liquid, a droplet was applied directly on top of the nostril and, by closing the mouth, the droplet was allowed to enter via the nostril into the nasal cavity. Spray dried Poly (I:C)-carrier powders were freshly prepared just prior to each experiment and administrated in 15 µl
liquid. Unformulated Poly (I:C) was administrated in phosphate
buffered saline (PBS) at a concentration of 1 mg/ml. Pretreatment was typically performed on day 2 or 3 before challenge. The mice were challenged on day 0 with 10 x LD₉₀ mouse adapted H1N1 PR (FLU PR 1600517) in 25 µl (high volume challenge) or with 1x LD₉₀ in 15 µl (low volume challenge). Following the challenge, mice were monitored daily for 14 days by measuring weight and behavior, mice were euthanized when weight loss was >20% compared to the day of challenge or when their behavior showed serious signs of illness.

## Claims

1. A microparticle consisting of polyinosinic acid, polycytidylic acid, pea starch, and water.

2. The microparticle of claim 1, wherein:
the average chain length of the polyinosinic acid and polycytidylic acid is approximately 300 base pairs to 6,000 base pairs;
the average molecular weight of the polyinosinic acid and polycytidylic acid is approximately 180 kDa to 3,600 kDa; and/or
the polyinosic acid and polycytidylic acid is present as a sodium salt.

3. The microparticle of claim 1 or 2, wherein the pea starch is hydroxypropylated pregelatinized pea starch.

4. The microparticle of any one of the preceding claims, wherein the ratio of polyinosinic acid and polycytidylic acid to pea starch in the microparticle is 1/200 (w/w) to 10/1 (w/w), 1/100 (w/w) to 1/1 (w/w), or 1/100 (w/w) to 1/5 (w/w).

5. The microparticle of claim 4, wherein the ratio of polyinosinic acid and polycytidylic acid to pea starch in the microparticle is 1/12 (w/w) to 1/9 (w/w).

6. A composition, comprising a plurality of microparticles according to any one of the preceding claims.

7. The composition of claim 6, wherein the microparticles of the plurality have a Dᵥ50 from 0.1 µm to 200 µm, 1 µm to 50 µm, 2 µm to 20 µm, or 10 µm to 20 µm.

8. The composition of claim 6 or 7, wherein the composition is a liquid, and the composition comprises an organic solvent, such as glycerol, ethanol, or a combination thereof.

9. The composition of claim 6 or 7, wherein the composition is a dry powder.

10. The composition of claim 9, wherein the particles are stable during storage at room temperature.

11. The composition of any one of claims 6 to 10, wherein the composition is suitable for nasal administration.

12. The composition of any one of claims 6 to 11, for use in a method of activating innate immune response.

13. The composition of any one of claims 6 to 12, for use in a method of treating or inhibiting a respiratory viral infection, or prophylaxing against a respiratory virus, such as a human rhinovirus or an influenza virus, optionally in a patient suffering from asthma and/or chronic obstructive pulmonary disease (COPD), optionally wherein the composition is formulated for nasal administration.

14. The composition for use of claim 13, wherein the respiratory viral infection is an infection of the upper respiratory tract, such as a human rhinovirus infection or an influenza virus infection.

15. A nasal delivery system, comprising the composition of any one of claims 6 to 14.

## Patentansprüche

1. Mikropartikel, bestehend aus Polyinosinsäure, Polycytidinsäure, Erbsenstärke und Wasser.

2. Mikropartikel nach Anspruch 1, wobei:
die durchschnittliche Kettenlänge der Polyinosinsäure und Polycytidinsäure ungefähr 300 Basenpaare bis 6000 Basenpaare beträgt,
das durchschnittliche Molekulargewicht der Polyinosinsäure und Polycytidinsäure ungefähr 180 kDa bis 3600 kDa beträgt und/oder
die Polyinosinsäure und Polycytidinsäure als ein Natriumsalz vorliegt.

3. Mikropartikel nach Anspruch 1 oder 2, wobei es sich bei der Erbsenstärke um hydroxypropylierte vorgelatinisierte Erbsenstärke handelt.

4. Mikropartikel nach einem der vorhergehenden Ansprüche, wobei das Verhältnis von Polyinosinsäure und Polycytidinsäure zu Erbsenstärke im Mikropartikel 1/200 (w/w) bis 10/1 (w/w), 1/100 (w/w) bis 1/1 (w/w) oder 1/100 (w/w) bis 1/5 (w/w) beträgt.

5. Mikropartikel nach Anspruch 4, wobei das Verhältnis von Polyinosinsäure und Polycytidinsäure zu Erbsenstärke im Mikropartikel 1/12 (w/w) bis 1/9 (w/w) beträgt.

6. Zusammensetzung, umfassend eine Vielzahl von Mikropartikeln nach einem der vorhergehenden Ansprüche.

7. Zusammensetzung nach Anspruch 6, wobei die Mikropartikel der Vielzahl einen Dᵥ50 von 0,1 µm bis 200 µm, 1 µm bis 50 µm, 2 µm bis 20 µm oder 10 µm bis 20 µm aufweisen.

8. Zusammensetzung nach Anspruch 6 oder 7, wobei es sich bei der Zusammensetzung um eine Flüssigkeit handelt und die Zusammensetzung ein organisches Lösungsmittel wie Glycerin, Ethanol oder eine Kombination davon umfasst.

9. Zusammensetzung nach Anspruch 6 oder 7, wobei es sich bei der Zusammensetzung um ein Trockenpulver handelt.

10. Zusammensetzung nach Anspruch 9, wobei die Partikel bei Lagerung bei Raumtemperatur stabil sind.

11. Zusammensetzung nach einem der Ansprüche 6 bis 10, wobei die Zusammensetzung für die nasale Verabreichung geeignet ist.

12. Zusammensetzung nach einem der Ansprüche 6 bis 11 zur Verwendung in einem Verfahren zur Aktivierung der angeborenen Immunreaktion.

13. Zusammensetzung nach einem der Ansprüche 6 bis 12 zur Verwendung bei einem Verfahren zur Behandlung oder zum Inhibieren einer viralen Infektion der Atemwege oder als Prophylaktikum gegen einen Virus der Atemwege wie einen humanen Rhinovirus oder einen Grippevirus, gegebenenfalls in einem an Asthma und/oder chronischer obstruktiver Lungenkrankheit (Chronic Obstructive Pulmonary Disease, COPD) leidenden Patienten, wobei die Zusammensetzung gegebenenfalls für die nasale Verabreichung formuliert ist.

14. Zusammensetzung zur Verwendung nach Anspruch 13, wobei es sich bei der viralen Infektion der Atemwege um eine Infektion der oberen Atemwege wie eine Infektion mit humanem Rhinovirus oder eine Infektion mit Grippevirus handelt.

15. Nasales Verabreichungssystem, umfassend die Zusammensetzung nach einem der Ansprüche 6 bis 14.

## Revendications

1. Microparticule constituée d'acide polyinosinique, d'acide polycytidylique, d'amidon de pois, et d'eau.

2. Microparticule selon la revendication 1, dans laquelle
la longueur de chaîne moyenne de l'acide polyinosinique et de l'acide polycytidylique va d'environ 300 paires de base à 6000 paires de base ;
le poids moléculaire moyen de l'acide polyinosinique et de l'acide polycytidylique va d'environ 180 kDa à 3600 kDa ; et/ou
l'acide polyinosinique et l'acide polycytidylique est présent sous forme d'un sel de sodium.

3. Microparticule selon la revendication 1 ou 2, dans laquelle l'amidon de pois est un amidon de pois prégélatinisé et hydroxypropylé.

4. Microparticule selon l'une quelconque des revendications précédentes, dans laquelle le rapport de l'acide polyinosinique et de l'acide polycytidylique à l'amidon de pois dans la microparticule va de 1/200 (p/p) à 10/1 (p/p), de 1/100 (p/p) à 1/1 (p/p), ou de 1/100 (p/p) à 1/5 (p/p).

5. Microparticule selon la revendication 4, dans laquelle le rapport de l'acide polyinosinique et de l'acide polycytidylique à l'amidon de pois dans la microparticule va de 1/12 (p/p) à 1/9 (p/p).

6. Composition, comprenant une pluralité de microparticules selon l'une quelconque des revendications précédentes.

7. Composition selon la revendication 6, dans laquelle les microparticules de la pluralité possèdent une Dᵥ50 allant de 0,1 µm à 200 µm, de 1 µm à 50 µm, de 2 µm à 20 µm, ou de 10 µm à 20 µm.

8. Composition selon la revendication 6 ou 7, la composition étant un liquide, et la composition comprenant un solvant organique, tel que le glycérol, l'éthanol, ou une combinaison de ceux-ci.

9. Composition selon la revendication 6 ou 7, la composition étant une poudre sèche.

10. Composition selon la revendication 9, dans laquelle les particules sont stables lors d'un stockage à température ambiante.

11. Composition selon l'une quelconque des revendications 6 à 10, la composition étant convenable pour une administration nasale.

12. Composition selon l'une quelconque des revendications 6 à 11, pour une utilisation dans une méthode d'activation de la réponse immunitaire innée.

13. Composition selon l'une quelconque des revendications 6 à 12, pour une utilisation dans une méthode de traitement ou d'inhibition d'une infection virale respiratoire, ou de prophylaxie contre un virus respiratoire, tel que le rhinovirus humain ou un virus influenza, éventuellement chez un patient souffrant d'asthme et/ou d'une bronchopneumopathie chronique obstructive (BPCO), éventuellement où la composition est formulée pour une administration nasale.

14. Composition pour une utilisation selon la revendication 13, dans laquelle l'infection virale respiratoire est une infection du tractus respiratoire supérieur, telle qu'une infection par un rhinovirus humain ou une infection par un virus influenza.

15. Système d'administration nasale, comprenant la composition selon l'une quelconque des revendications 6 à 14.
